# EUROPEAN PATENT APPLICATION

(11) **EP 4 140 554 A1**
(43) Date of publication of application: **01.03.2023**
(21) Application number: 22190357.8
(22) Date of filing: 15.08.2022
(51) Int. Cl.: A63F 13/655, A63F 13/212, A63F 13/213, A63F 13/218, A63F 13/285, A63F 13/428, A63F 13/537, A63B 71/06, A63F 13/67, G06F 3/01, G16H 20/30

(54) **EXTENDED REALITY SYSTEMS, APPARATUS, AND METHODS FOR MUSCULOSKELETAL ERGONOMIC IMPROVEMENT**

(30) Priority: 25.08.2021 US 202117412049
(71) Applicant: The Boeing Company, Chicago, IL 60606-2016 (US)
(72) Inventor: LAUGHLIN, Brian D, Chicago, 60606-2016 (US); GEORGESON, Gary E, Chicago, 60606-2016 (US)
(74) Representative: Boult Wade Tennant LLP

(57) **Abstract**

Extended reality systems, apparatus, and methods for musculoskeletal ergonomic improvement are disclosed. An example apparatus includes an avatar generator to generate an avatar based one or more properties of a user; an avatar position analyzer to determine a first ergonomic form for a movement based on the one or more properties of the user, the avatar generator to cause an output device to display the avatar in the first ergonomic form; and a feedback generator to determine a second form associated with movement of the user based on sensor data collected via one or more sensors associated with the user; generate a graphical representation of the user in the second form; and cause the output device to display the graphical representation of the user with the avatar.

## Description

### FIELD OF THE DISCLOSURE

This disclosure relates generally to ergonomics and, more particularly, to extended reality systems, apparatus, and methods for musculoskeletal ergonomic improvement.

### BACKGROUND

Extended reality devices such as augmented reality headsets can generate environments that combine reality with digital features. For instance, a user wearing an augmented reality headset can be guided to perform an action in the real world via information provided in a digital format, where the digital content appears in the user's environment.

### SUMMARY

An example apparatus includes an avatar generator to generate an avatar based one or more properties of a user; an avatar position analyzer to determine a first ergonomic form for a movement based on the one or more properties of the user, the avatar generator to cause an output device to display the avatar in the first ergonomic form; and a feedback generator to determine a second form associated with movement of the user based on sensor data collected via one or more sensors associated with the user; generate a graphical representation of the user in the second form; and cause the output device to display the graphical representation of the user with the avatar.

An example system includes a first sensor and an extended reality coach controller to execute a neural network model to generate an avatar illustrating a first ergonomic position for a movement; cause an extended reality device to output the avatar; determine a second position of a body part of a user based on first sensor data generated by the first sensor; perform a comparison of the first ergonomic position and the second position; and cause the extended reality device to output graphical feedback based on the comparison

An example non-transitory computer readable medium comprising instructions that, when executed by at least one processor, cause the at least one processor to generate an avatar based one or more properties of a user; determine a first ergonomic form for a movement based on the one or more properties of the user; cause an output device to display the avatar in the first ergonomic form; determine a second form associated with movement of the user based on sensor data collected via one or more sensors associated with the user; generate a graphical representation of the user in the second form; and cause the output device to display the graphical representation of the user with the avatar.

An example method includes generating an avatar based one or more properties of a user; determining a first ergonomic form for a movement based on the one or more properties of the user; causing an output device to display the avatar in the first ergonomic form; determining a second form associated with movement of the user based on sensor data collected via one or more sensors associated with the user; generating a graphical representation of the user in the second form; and causing the output device to display the graphical representation of the user with the avatar

### BRIEF DESCRIPTION OF THE DRAWINGS

FIG. 1 illustrates an example system constructed in accordance with teachings of this disclosure and including data collection device(s) for collecting ergonomic data associated with a user and an extended reality (XR) coach controller for generating an avatar or digital coach to be presented to the user via an extended reality device to demonstrate ergonomic form(s) for movement(s) to be performed by the user.
FIG. 2 is a block diagram of the example system of FIG. 1 including an example implementation of the XR coach controller of FIG. 1 and a computing system for training neural network(s) to generate model(s) for use by the XR coach controller in determining the ergonomic forms to be demonstrated by the avatar.
FIG. 3 illustrates an example avatar or digital coach generated by the example XR coach controller of FIGS. 1 and/or 2 in accordance with teachings of this disclosure.
FIG. 4 illustrates example graphical feedback generated by the example XR coach controller of FIGS. 1 and/or 2 with respect to user movements relative to an avatar.
FIG. 5 is a flowchart representative of example machine readable instructions that, when executed by the neural network computing system of FIG. 2, cause the computing system to train a neural network to determine ergonomic forms to be demonstrated by an avatar.
FIG. 6 is a flowchart representative of example machine readable instructions that, when executed by the XR coach controller of FIGS. 1 and/or 2, cause the XR coach controller to generate an avatar or digital coach to demonstrate ergonomic forms for movements to be performed by the user.
FIG. 7 is a block diagram of an example processing platform structured to execute the instructions of FIG. 5 to implement the example neural network computing system of FIG. 2.
FIG. 8 is a block diagram of an example processing platform structured to execute the instructions of FIG. 6 to implement the example XR coach controller of FIGS. 1 and/or 2.

The figures are not to scale. In general, the same reference numbers will be used throughout the drawing(s) and accompanying written description to refer to the same or like parts.

Unless specifically stated otherwise, descriptors such as "first," "second," "third," etc. are used herein without imputing or otherwise indicating any meaning of priority, physical order, arrangement in a list, and/or ordering in any way, but are merely used as labels and/or arbitrary names to distinguish elements for ease of understanding the disclosed examples. In some examples, the descriptor "first" may be used to refer to an element in the detailed description, while the same element may be referred to in a claim with a different descriptor such as "second" or "third." In such instances, it should be understood that such descriptors are used merely for identifying those elements distinctly that might, for example, otherwise share a same name.

### DETAILED DESCRIPTION

A user interacting with an extended reality device, such as an augmented reality headset, can be guided to perform an action in the real world via information provided in a digital format, where the digital content appears in the user's environment. The digital content can include an avatar, or a graphical representation of a person or character.

An individual may experience a musculoskeletal injury (e.g., an injury to muscle(s), nerve(s), and/or joint(s) of the individual's body) while performing tasks. Such injuries can stem from conditions in a work environment and/or a manner in which the activities are performed. For instance, performing repetitive tasks, lifting heavy objects, and/or other types of overuse or overexertion activities can cause musculoskeletal injuries that, in addition to causing pain, may affect worker productivity. Efforts to reduce musculoskeletal injuries are often not addressed until the worker is experiencing pain.

Disclosed herein are example systems, apparatus, and methods that generate an avatar or digital coach to demonstrate reference, correct, or otherwise optimal ergonomic form(s) for performing movement(s) in connection with a movement and/or task to be performed by a user, such as lifting a box, installing a component, etc. Examples disclosed herein generate the avatar based on features of the user, such as height, age, gender, etc. of the user. Examples disclosed herein execute neural network model(s) to determine reference or optimal ergonomic forms for the user in performing the movement(s), such as body part position(s), posture(s), muscle tension level(s), speed(s) at which the movement(s) are to performed, etc. Examples disclosed herein implement the neural network models based on properties of the user and/or users with similar properties as the user of interest to develop customized recommendations for the user based on the user's body type, musculoskeletal injury history, etc. to provide ergonomic form recommendations to the user when performing movement(s) associated with the task.

Examples disclosed herein provide the user with feedback indicative of a performance of the user relative to the ergonomic forms presented by the avatar. Some examples disclosed herein analyze sensor data collected from the user and/or the environment in which the user is located to determine positions of body parts and/or other movement characteristics of the user. Examples disclosed herein compare the positions and/or other movement characteristics of the user to the ergonomic forms determined by the neural network analysis. The sensor data can include, for instance, position data (e.g., accelerometer data), muscle strain data, image data, etc. In some examples, the feedback includes graphical representations of the user presented with the avatar (e.g., a graphical representation of the user overlaying an image of the avatar). Such feedback shows differences between the user's form and the reference or optimal form presented by the avatar. Examples disclosed herein use extended reality to provide customized recommendations and feedback to guide the user in performing movements in accordance with recommended ergonomic forms.

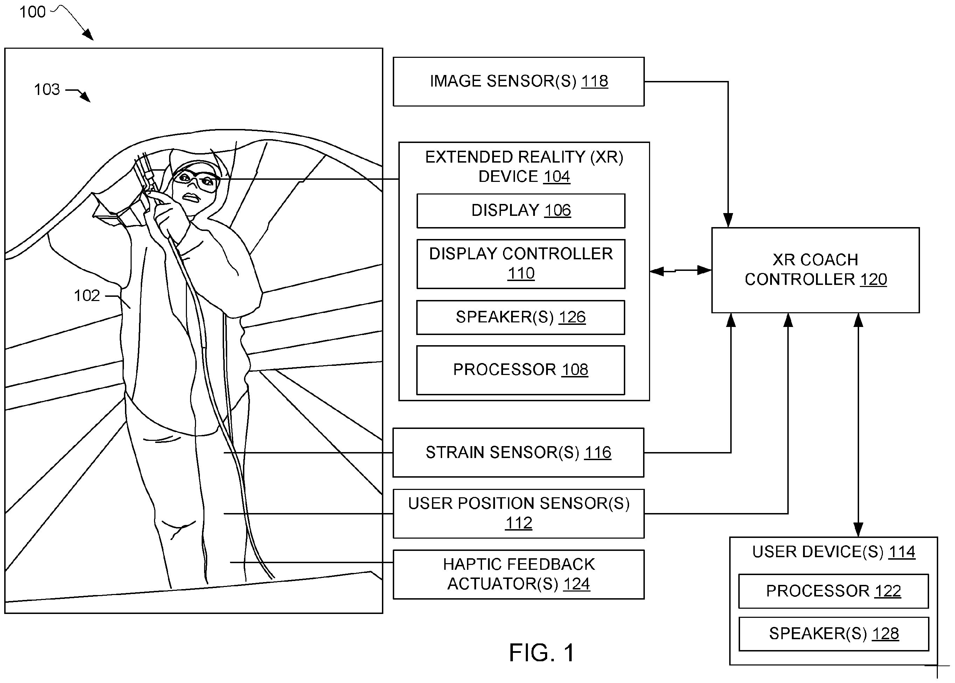

FIG. 1 illustrates an example system 100 constructed in accordance with teachings of this disclosure for providing a user 102 with ergonomic feedback with respect to movement(s) performed by the user 102 when completing a task in an environment 103 using extended reality. As shown in FIG. 1, the user 102 (the terms "user" and "subject" are used interchangeably herein and both refer to a human being) is performing a task in the environment 103 that involves the user 102 installing component(s) of an aircraft. In particular, the user 102 is installing the component(s) while standing, where arms of the user 102 are raised to reach the component(s) located above the shoulders of the user 102. The user 102 may perform other task(s) and associated movement(s) than the example shown in FIG. 1. Also, the environment 103 can differ from the example shown in FIG. 1.

The example system 100 of FIG. 1 includes an extended reality (XR) device 104. In the example of FIG. 1, the XR device 104 includes eyeglasses worn by the user 102. However, the XR device 104 can include other wearable devices such as a mask, goggles, etc.

The example XR device 104 includes a display 106. As disclosed herein, the display 106 provides means for presenting extended reality content to the user 102. The extended reality content can include, for example, virtual reality content, augmented reality content, and/or mixed reality content depending on, for example, the type of XR device 104 (e.g., a VR headset) and/or the type of content to be presented (e.g., mixed reality content to facilitate training stimulations in the real-world with virtual guidance). In the example of FIG. 1, the extended reality content includes an avatar that demonstrates to the user 102 how to perform movement(s) with recommended, reference, proper, or otherwise optimal ergonomic form when performing a given task. Thus, in examples disclosed herein, the avatar can serve as a digital coach for the user 102. The XR device 104 includes a processor 108. In some examples, the processor 108 is coupled to (e.g., mounted to, carried by) the XR device 104 (e.g., a frame of the XR device 104). In other examples, the processor 108 is separate from the XR device 104. The XR device 104 includes a display controller 110 to output signals to cause the display 106 to display the extended reality content (e.g., images of the avatar) based on instructions from, for instance, the processor 108.

The example system 100 includes one or more sensors to collect data from the user 102 and/or the environment 103 with respect to movement(s) performed by the user 102. In particular, the sensor(s) collect data associated with the user 102 during use of the XR device 104 (e.g., during presentation of and/or interaction with the avatar or digital coach). For example, the sensor(s) can include user position sensors 112 to generate data indicative of movement of one or more portions of the body of the user 102. The user position sensor(s) 112 can include motion captures sensor(s), accelerometer(s), etc. to output data indicative of change(s) in position of one or more portion(s) of the body of the user 102 (e.g., arms, legs, wrists, etc.). In some examples, the user position sensor(s) 112 include weight, pressure, and/or load sensor(s) to detect changes in weight transfer between one or more portions of the user's body (e.g., between the user's feet). The user position sensor(s) 112 can be carried by the user 102, by the XR device 104, and/or by other user device(s) 114 (e.g., a smartwatch, a smartphone, etc.) carried by the user 102 and/or located in the environment 103

The example system 100 includes one or more strain sensor(s) 116 to detect strain and/or stress on joint(s) of the user 102 and/or with respect to the muscle(s) of the user 102. The strain sensor(s) 116 can include electromyography (EMG) sensor(s) worn by the user 102 to detect muscle tension. In some examples, the strain sensor(s) 116 include sensor(s) to detect skin and/or muscle temperature, which are indicative of muscle activity. In some examples, the strain sensor(s) 116 include fabric sensing wearable(s). The fabric sensing wearable(s) include wearable fabrics (e.g., a shirt or other garment) that include sensor(s) to output data indicative of strain on the muscle(s) and/or skeleton (e.g., joint(s)) of the user 102. For example, motion-sensing fabrics can include pressure and/or strain sensor(s) that output signal(s) in response to changes in pressure and/or deformation of the sensor(s) during movement by the user 102. The strain sensor(s) 116 can be carried by the user 102 and/or by user device(s) 104, 114 associated with the user 102.

The example system 100 includes image sensor(s) 118 (e.g., camera(s)) to generate image data of the user 102 in the environment 103. For example, the image sensor(s) 118 may be located in a room in the environment 103 in which the user 102 is performing the task(s) (e.g., in the manufacturing facility of FIG. 1). In the example of FIG. 1, the image sensor(s) 118 capture image(s) of the user 102 from multiple angles and/or views in the environment 103. For example, a first image sensor 118 (e.g., a first camera) can generate image data including a front view of the user 102 and a second image sensor 118 (e.g., a second camera) can generate image data including a side view of the user 102. In some examples, the image sensor(s) 118 include infrared camera(s) that detect changes in a temperature of a skin of the user 102 due to muscle activity.

The example system 100 can include other types of sensors than the example sensors 112, 116, 118 disclosed herein. Also, in some examples, the system 100 includes fewer types of sensor(s).

In the example of FIG. 1, the signals output by the user position sensor(s) 112, the strain sensor(s) 116, and the image sensor(s) 118 are transmitted to an extended reality (XR) coach controller 120. In some examples, the XR coach controller 120 is implemented by instructions executed on the processor 108 of the XR device 104 and/or by respective processor(s) 122 of the other user device(s) 114. In other examples, the XR coach controller 120 is implemented by one or more cloud-based device(s) such as one or more servers, processors, and/or virtual machines. In other examples, some of the analysis performed by the XR coach controller 120 is implemented by the cloud-based device(s) and other parts of the analysis are implemented by the processor(s) 108, 122 of the one or more user device(s) 104, 114.

In some examples, as represented in FIG. 1, the user position sensor(s) 112, the strain sensor(s) 116, and/or the image sensor(s) 118 are in (e.g., direct) communication with the XR coach controller 120 via wireless connection(s) (Bluetooth^{®}, WiFi connections with the sensor(s) 112, 116, 118 and/or with the user device(s) carrying the sensor(s) 112, 116, 118).

In other examples, the signals output by one or more of the user position sensor(s) 112, the strain sensor(s) 116, and/or the image sensor(s) 118 are transmitted to, for instance, the processor 108 of the XR device 104 for processing before being transmitted to the XR coach controller 120 (i.e., in examples where the XR coach controller 120 is implemented by processor(s) and/or cloud-based device(s) different than the on-board processors 108 of the XR device 104). For example, the processor 108 of the XR device 104 can perform operations such as removing noise from the signal data, and/or converting the signal data from analog to digital data. In such examples, the on-board processor 108 of the XR device 104 is in communication (e.g., wireless communication) with the XR coach controller 120. Additionally or alternatively, the pre-processing can be performed by the processor(s) 122 of the other user device(s) 114.

In some examples, the XR coach controller 120 receives sensor data from the user position sensor(s) 112, the strain sensor(s) 116, and/or the image sensor(s) 118; from the processor 108 of the XR device 104; and/or from the processor(s) 122 of the other user device(s) 114 in real time/substantially real-time (as used herein "real time" and "substantially real time" refers to occurrence in a near instantaneous manner (e.g., +/- 1 second) recognizing there may be real world delays for computing time, transmission, etc.). In other examples, the XR coach controller 120 receives the sensor data at a later time (e.g., periodically and/or aperiodically based on one or more settings but sometime after the activity that caused the sensor data to be generated, such as movement by the user 102, has occurred (e.g., seconds later)). If the data has not already been processed, the XR coach controller 120 can perform one or more operations on the data from the sensor(s) 112, 116, 118 such as filtering the raw signal data, removing noise from the signal data, and/or converting the signal data from analog to digital data.

In the example of FIG. 1, the XR coach controller 120 generates an avatar or digital coach and causes the avatar to be output by the XR device 104 for presentation to the user 102 (e.g., via augmented reality). In the example of FIG. 1, the avatar is a digitally animated character that provides instructions to the user 102 for performing movement(s) in connection with a task while the user 102 is performing the movement. For example, the avatar generated by the XR coach controller 120 can demonstrate ergonomically correct forms when lifting a box to provide the user 102 with visual (i.e., graphical) instructions for lifting a box. In such examples, the avatar may perform actions such as bending its knees before lifting the box.

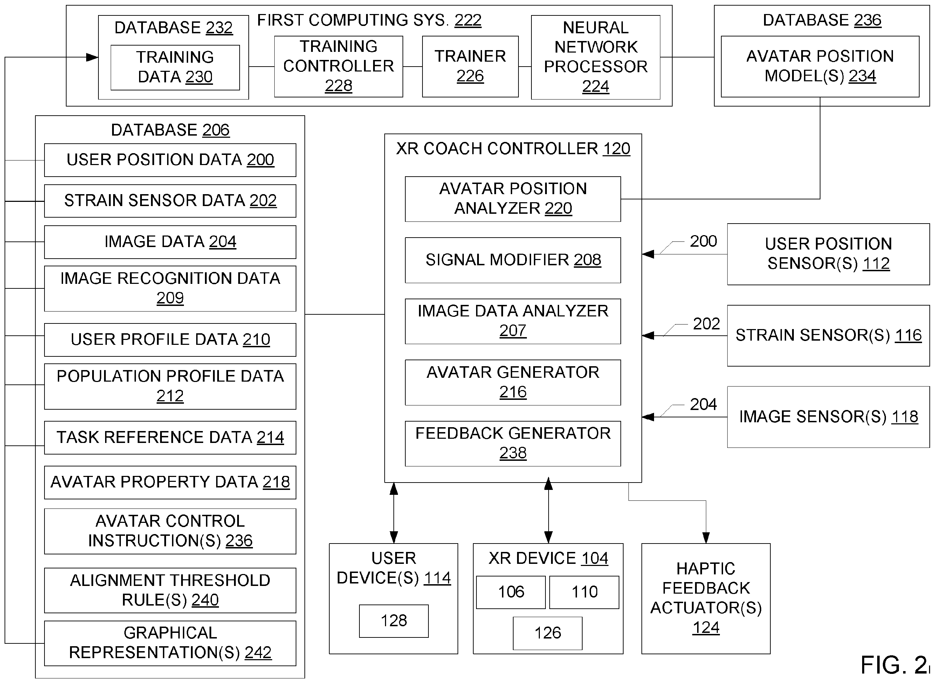

In the example of FIG. 1, the XR coach controller 120 generates the avatar based on one or more properties of the user 102, such as a gender, height, weight, etc. of the user 102. The user properties can be provided via user inputs and stored in a user profile (e.g., the user profile data 210 discussed in connection with FIG. 2) that is used to generate an avatar exhibiting or representing similar properties as the user 102. In other examples, the avatar is based on other user(s) and/or includes other content (e.g., represents a non-human character such as an animal). The XR coach controller 120 causes the display controller 110 of the XR device 104 to display the avatar via the display 106 of the XR device 104.

In some examples disclosed herein, the XR coach controller 120 implements neural network model(s) to cause the avatar to illustrate ergonomic forms including posture(s), position(s), orientation(s), range(s) of motion, muscle tension level(s), speed(s), etc. for performing movement(s) to promote and/or preserve musculoskeletal integrity. In some examples disclosed herein, the ergonomic forms are based on properties of the user 102 and/or other individuals sharing properties with the user 102. For instance, the neural network model(s) can be generated using information for the user 102 and/or other users based on properties such as age, gender, physical body shape, weight, previous medical history (e.g., injuries, conditions such as arthritis). The user properties can be provided as user inputs at one or more of the XR device 104 or the other user device(s) 114. In some examples, the neural network(s) are trained based on image data, position sensor data, and/or strain sensor data generated for the user 102 and/or other users. The neural network model(s) can be generated for particular tasks such as lifting a box, installing a component located overhead, and/or other tasks defined based on the environment 103, the role of the user 102, etc. As a result of the neural network analysis, the XR coach controller 120 controls the avatar to demonstrate reference, correct, or optimal ergonomically form(s) for performing a task that are customized for the user 102.

In the example of FIG. 1, the movement(s) performed by avatar as result of the neural network analysis performed by the XR coach controller 120 represent reference (e.g., optimal) movement(s) and/or ergonomic forms that promote and/or preserve musculoskeletal integrity. The movement(s) demonstrated by the avatar account for user properties and/or abilities by customizing the ergonomic form recommendations based on user profile data and/or data (e.g., real-time data) generated by the user position sensor(s) 112, the strain sensor(s) 116, and/or the image sensor(s) 118 while the user 102 is performing movements. For example, for a movement such as placing an item on a shelf, the XR coach controller 120 may generate a first ergonomic form recommendation for a first user having a first height that indicates that the first user can reach the shelf without assistance. For instance, the first ergonomic form recommendation may advise the user how to lift an object above his or her head to place the item on the shelf without overreaching. The XR coach controller 120 can monitor and/or adjust the ergonomic form recommendation based on, for example, data from the strain sensors 116. For a second user having a second height that indicates that the second user is not able to reach the shelf, the XR coach controller 120 may generate a second ergonomic form recommendation that advises the second user how to ascend and descend a ladder with proper form to reduce stress on the second user's legs and/or back. Some examples disclosed herein also provide feedback to the user 102 with respect to the movement(s) performed by the user 102 relative to the movement(s) illustrated by the avatar to further encourage the user 102 to perform ergonomically correct movement(s).

The example XR coach controller 120 of FIG. 1 analyzes the data from the user position sensor(s) 112, the strain sensor(s) 116, and/or the image sensor(s) 118 to determine the ergonomic form(s) (e.g., posture(s), position(s), orientation(s), range(s) of motion, muscle tension level(s), speed(s), etc.) for one or more body parts of the user 102. Based on the analysis of the user position data, the strain sensor data, and/or the image data, the XR coach controller 120 determines whether the ergonomic forms(s) of the user 102 are consistent with or substantially consistent with (e.g., match, substantially match, align with) the reference ergonomic forms(s) illustrated by the avatar. For example, the XR coach controller 120 can analyze the image data generated by the image sensor(s) 118 to detect position(s) of one or more body parts of the user 102. For example, the XR coach controller 120 analyze the image data using image recognition analysis and/or other machine learning image analysis to identify the locations of joints (e.g., knee joint, elbow joint, wrist joint) of the user when the user is in a particular pose as captured in the image. Based on the locations of the joints or keypoints, the XR coach controller 120 can generate a skeletal representation of the user in a particular position. In other example, the XR coach controller 120 can determine position(s) of the body part(s) of the user 102 using data from the user position sensor(s) 112. In some examples, the XR coach controller 120 compares strain exerted by the user 102 as detected by the strain sensor(s) 116 to reference strain levels determined by the XR coach controller. The XR coach controller 120 generates feedback to be provided to the user 102 via the XR device 104 and/or the other user device(s) 114 to inform the user 102 whether the user 102 is performing the movement(s) correctly (i.e., as demonstrated by the avatar) or is preforming the movement(s) incorrectly and, thus, may be at a risk of musculoskeletal injury.

In some examples, the feedback generated by the XR coach controller 120 includes graphical or visual representation(s) of the user. The graphical representation(s) can illustrate the position(s) of the body part(s) of the user 102 (e.g., an arm of the user 102) relative to corresponding portion(s) of the avatar (e.g., an arm of the avatar) presented via the display 106 of the XR device 104. For instance, a graphical representation of the body of the user can be presented as overlaying an image of the avatar to enable a comparison of the alignment of the user 102 with the avatar in a particular position.

In some examples, the XR coach controller 120 causes a graphical feature of the avatar to be adjusted based on the comparison of the ergonomic form of the user to the reference ergonomic form. For example, the XR coach controller 120 can cause a color of the avatar to change to a first color (e.g., green) to provide visual feedback to the user 102 when the XR coach controller 120 determines that the position(s) of the body part(s) of the user 102 align or substantially align with corresponding portion(s) of the avatar. The XR coach controller 120 can cause a color of the avatar to change to a second color (e.g., red) to alert the user 102 when the XR coach controller 120 determines that the position(s) of the body part(s) of the user 102 do not align or do not substantially align with corresponding portion(s) of the avatar.

The feedback generated by the XR coach controller 120 can additionally or alternatively include other types of feedback involving the avatar. For example, the XR coach controller 120 can cause other types of graphical feedback to be presented via the XR device 104. For instance, the XR coach controller 120 can cause the avatar to perform actions such as clapping in response to a determination based on data from the sensor(s) 112, 116, 118 that the user 102 is performing a movement with correct posture. The XR coach controller 120 can cause other types of content (e.g., video content) to be presented via the XR device 104 to provide feedback to the user 102, such as a check mark that is to be displayed when the XR coach controller 120 determines that the user 102 performed the movement with correct posture.

In some examples, the feedback generated by the XR coach controller 120 includes audio feedback to be output via, for example, the speaker(s) 126 of the extended reality device 104 and/or speaker(s) 128 of the other user device(s) 114. The audio feedback can include instructions with respect to performing the movement(s) (e.g., "bend your knees before lifting the box") and/or feedback regarding whether the user 102 performed the movement with proper form. Additionally or alternatively, the feedback generated by the XR coach controller 120 can include textual instructions with respect to adjustments to the user's form that are displayed via, for instance, the display 106 of the XR device 104.

In some examples, feedback from the XR coach controller 120 is provided via haptic feedback actuator(s) 124. The haptic feedback actuator(s) 124 can be carried by, for example, the user 102, the XR device 104, the other user device(s) 114 (e.g., a smartphone carried by the user 102), etc. In some examples, the XR coach controller 120 instructs the haptic feedback actuator(s) 124 to provide haptic feedback output(s) (e.g., vibrations) while the avatar is being presented to make the user 102 aware of his or her posture, speed of movement, tension exerted, etc. relative to the avatar. In other examples, the XR coach controller 120 causes the haptic feedback to be output independent of the presentation of the avatar and in response to, for example, detection of movement by the user 102.

In some examples, the other user device(s) 114 include user device(s) (e.g., electronic tablets, smartphones, laptops) associated with the user 102 and/or a third party who is authorized to receive report(s), alert(s), etc. with respect to the analysis of the sensor data, performance of movement(s) of the user 102 relative to the recommendations presented by the avatar, etc. The third party can include, for example, a medical professional. In such examples, the XR coach controller 120 can transmit the data collected by the sensor(s) 112, 116, 118 and/or results of analyses thereof for display at the output device(s) 114. Thus, the authorized third party can track changes in the user 102 and/or other users with respect to ergonomic performance over time.

FIG. 2 is a block diagram of an example implementation of the extended reality (XR) coach controller 120 of FIG. 1. As mentioned above, the example XR coach controller 120 is constructed to generate an avatar (e.g., a digital coach) to be presented via an XR device (e.g., the XR device 104 of FIG. 1) to provide ergonomic feedback to a user (e.g., the user 102 of FIG. 1) with respect to positioning one or more portions of the user's body when performing movement(s). In the example of FIG. 2, the XR coach controller 120 is implemented by one or more processor(s) of user device(s) (e.g., the XR device 104, the other user device(s) 114 of FIG. 1) and/or cloud-based device(s) (e.g., server(s), processor(s), and/or virtual machine(s)). In some examples, some of the analysis is implemented by the XR coach controller 120 via a cloud-computing environment and one or more other parts of the analysis is implemented by processor(s) of one or more user device(s).

In the example of FIG. 2, user position data 200 generated by the user position sensor(s) 112, strain sensor data 202 collected from the user 102 by the strain sensor(s) 116, and/or image data 204 generated by the image sensor(s) 118 is transmitted to the XR coach controller 120. In some examples, the data 200, 202, 204 is transmitted to the XR coach controller 120 by the sensor(s) 112, 116, 118. In other examples, the data 200, 202, 204 is transmitted to the XR coach controller 120 by the processor 108 of the XR device 104 and/or the processor(s) 122 of the other user device(s) 114 after, for example, pre-processing of the data 200, 202, 204 is performed at those devices. This transmission of the data 200, 202, 204 to the XR coach controller 120 may be in real time/substantially in real time (e.g., as the data is gathered), periodically (e.g., every five seconds), and/or may be aperiodic (e.g., based on factor(s) such as an amount of data collected, memory storage capacity usage, whether the user 102 has performed a movement, etc.). Although examples disclosed herein generally refer to the data 200, 202, 204, in some examples, not all types of the data 200, 202, 204 is collected and/or used in the analyses performed herein.

In the example of FIG. 2, a database 206 provides means for storing the user position data 200, the strain sensor data 202, and the image data 204. In the example of FIG. 2, the database 206 can store the data 200, 202, 204 over time. In some examples, the XR coach controller 120 includes the database 206. In other examples, the database 206 is located external to the XR coach controller 120 in a location accessible to the XR coach controller 120 as shown in FIG. 2.

The example XR coach controller 120 includes a signal modifier 208. The signal modifier 208 can perform operations to modify the sensor data 200, 202, 204 from the sensor(s) 112, 116, 118 to, for example, filter the data, convert time domain audio data into the frequency domain (e.g., via Fast Fourier Transform (FFT) processing) for spectral analysis, etc. In some examples, the signal modifier 208 processes the sensor data 200, 202, 204 if pre-processing of the data has not otherwise been performed at the XR device 104 and/or the other user device(s) 114. In some examples, the data 201, 202, 204 undergoes modification(s) by the signal modifier 208 before being stored in the database 206.

The example XR coach controller 120 includes an image data analyzer 207. The image data analyzer 207 analyzes the image data 204 generated by the image sensor(s) 118 using image recognition analysis to, for example, identify the user 102 in the image data, detect locations of body part(s) of the user 102, etc. In some examples, the image data analyzer 207 identifies the body part(s) of the user 102 in the image data 204 using keypoint detection, where the keypoints represent joints of the user 102. The results of the image recognition analysis performed by the image data analyzer 207 (e.g., keypoint locations) are stored in the database 206 as image recognition data 209.

In the example of FIG. 2, user profile data 210 is received by the XR coach controller 120 and stored in the database 206. The user profile data 210 can be provided as user input(s) at the XR device 104 and/or the other user device(s) 114. The user profile data 210 can include characteristics and/or properties of the user 102, such as gender, age, weight, height, etc. of the user 102. The user profile data 210 can include other types of data such as a job position of the user 102 (e.g., to indicate a nature of tasks and/or movements to be performed), a medical history for the user 102 (e.g., previous injuries to body part(s) of the user 102, conditions such as arthritis), etc.

The user profile data 210 can define task(s) to be performed by the user 102 for which the avatar is to provide instructions with respect to reference or optimal ergonomic movements. For example, the task(s) can be associated with a job of the user 102 such as installing a component while on a ladder, loading a truck with inventory, etc.

In some examples, the database 206 stores population profile data 212. The population profile data 212 can include data associated with individuals in a population sharing one or more characteristics with the user 102, such as an average height of an individual based on gender and age. The population profile data 212 can include average muscle forces exerted by users at different ages, with different health conditions, etc. The population profile data 212 can include average distances between joints in users of a certain height. The population profile data 212 can be defined by user input(s) and can include other types of data than the examples disclosed herein.

In example of FIG. 2, task reference data 214 is stored in the database 206. The task reference data 214 defines tasks and associated movements for performing the tasks. In the example of FIG. 2, the movements defined in the task reference data 214 include ergonomically correct form(s) (e.g., posture(s), position(s), orientation(s), muscle tension level(s), etc.) that serve as reference data for determining form(s) to be performed by the user 102. For example, the task reference data 214 can include movements in connection with lifting a box to reduce back strain (e.g., bend knees, keep back straight, etc.). The ergonomically correct form(s) included in the task reference data 214 can be defined based on, for example, known medical data. The task reference data 214 can be provided to the XR coach controller 120 via one or more user input(s).

The example XR coach controller 120 of FIG. 2 includes an avatar generator 216. The avatar generator 216 generates an avatar or digital coach based on the user profile data 210. For example, the avatar generator 216 can generate an avatar having the same gender as the user 102 and representing a similar height. Data associated with the avatar generated by the avatar generator 216 is stored in the database 206 as avatar property data 218.

As disclosed herein, the avatar generated by the avatar generator 216 is controlled to perform movement(s) with reference or optimal ergonomic form(s) to guide the user 102 in performing movements (e.g., in connection with a task) in ergonomically correct form. The example XR coach controller 120 of FIG. 2 includes an avatar position analyzer 220. In the example of FIG. 2, the avatar position analyzer 220 executes neural network model(s) to determine position(s), posture(s), movement(s), range(s) of motion, speed(s), force exerted, and/or other properties or forms associated with movement(s) to be illustrated by an avatar when demonstrating task(s) such as a lifting a box, installing a component overhead, etc. In the example of FIG. 2, the properties or forms associated with the movement(s) to be illustrated by the avatar are customized for the user 102 based on the neural network analysis. The avatar generator 216 executes the instructions generated by the avatar position analyzer 220 to control the avatar to perform the movement(s) with respect to ergonomic forms as determined by the neural network analysis.

Artificial intelligence (AI), including machine learning (ML), deep learning (DL), and/or other artificial machine-driven logic, enables machines (e.g., computers, logic circuits, etc.) to use a model to process input data to generate an output based on patterns and/or associations previously learned by the model via a training process. For instance, the model may be trained with data to recognize patterns and/or associations and follow such patterns and/or associations when processing input data such that other input(s) result in output(s) consistent with the recognized patterns and/or associations.

In general, implementing a ML/AI system involves two phases, a learning/training phase and an inference phase. In the learning/training phase, a training algorithm is used to train a model to operate in accordance with patterns and/or associations based on, for example, training data. In general, the model includes internal parameters that guide how input data is transformed into output data, such as through a series of nodes and connections within the model to transform input data into output data. Additionally, hyperparameters are used as part of the training process to control how the learning is performed (e.g., a learning rate, a number of layers to be used in the machine learning model, etc.). Hyperparameters are defined to be training parameters that are determined prior to initiating the training process.

Different types of training may be performed based on the type of ML/AI model and/or the expected output. For example, supervised training uses inputs and corresponding expected (e.g., labeled) outputs to select parameters (e.g., by iterating over combinations of select parameters) for the ML/AI model that reduce model error. As used herein, labelling refers to an expected output of the machine learning model (e.g., a classification, an expected output value, etc.). Alternatively, unsupervised training (e.g., used in deep learning, a subset of machine learning, etc.) involves inferring patterns from inputs to select parameters for the ML/AI model (e.g., without the benefit of expected (e.g., labeled) outputs).

Training is performed using training data. In examples disclosed herein, the training data originates from previously generated sensor data (e.g., user position data, strain sensor data such as EMG data or fabric stretch sensor data, image data of user(s) performing different movement(s), user parameter data (e.g., weight, gender), motion capture sensor data, etc.) for movement associated with reference or correct ergonomic forms. Because supervised training is used, the training data is labeled.

Once training is complete, the model is deployed for use as an executable construct that processes an input and provides an output based on the network of nodes and connections defined in the model. The model(s) are stored at one or more databases (e.g., the database 236 of FIG. 2). The model may then be executed by the avatar position analyzer 220 of the example XR coach controller 120 of FIG. 2.

Once trained, the deployed model may be operated in an inference phase to process data. In the inference phase, data to be analyzed (e.g., live data) is input to the model, and the model executes to create an output. This inference phase can be thought of as the AI "thinking" to generate the output based on what it learned from the training (e.g., by executing the model to apply the learned patterns and/or associations to the live data). In some examples, input data undergoes pre-processing before being used as an input to the machine learning model. Moreover, in some examples, the output data may undergo post-processing after it is generated by the AI model to transform the output into a useful result (e.g., a display of data, an instruction to be executed by a machine, etc.).

In some examples, output of the deployed model may be captured and provided as feedback. By analyzing the feedback, an accuracy of the deployed model can be determined. If the feedback indicates that the accuracy of the deployed model is less than a threshold or other criterion, training of an updated model can be triggered using the feedback and an updated training data set, hyperparameters, etc., to generate an updated, deployed model.

Referring to FIG. 2, the example system 100 includes a first computing system 222 to train a neural network to generate instructions for controlling an avatar to demonstrate reference or optimal ergonomic forms (e.g., posture(s), position(s), orientation(s), range(s) of motion, speed(s), etc.) when performing movement(s) based properties of the user 102. The example first computing system 222 includes a neural network processor 224. In examples disclosed herein, the neural network processor 224 implements a neural network.

The example first computing system 222 of FIG. 2 includes a neural network trainer 226. The example neural network trainer 226 of FIG. 2 performs training of the neural network implemented by the neural network processor 224.

The example first computing system 222 of FIG. 2 includes a training controller 228. The example training controller 228 instructs the neural network trainer 226 to perform training of the neural network based on training data 230. In the example of FIG. 2, the training data 230 used by the neural network trainer 226 to train the neural network is stored in a database 232.

In the example of FIG. 2, the training data 230 includes, for example, image data of user(s) performing movement(s) with ergonomically correct form(s), position data including locations of body part(s) of user(s) in ergonomically correct form(s) when performing movement(s), strain sensor data with respect to muscle tension during certain tasks, etc. The training data 230 can be for particular task(s) (e.g., lifting a box) and/or for particular portion(s) of the body (e.g., shoulders, back, neck, legs). The training data can include the task reference data 214 that includes reference (e.g., optimal) movement(s) associated with different tasks.

In the example of FIG. 2, the training data 230 is particular for the user 102 and/or users with properties similar to the user 102 (e.g., gender, height, weight, etc.). In some examples, the training data 230 includes the user profile data 210. In some examples, the training data 230 includes the user position data 200, the strain sensor data 202, the image data 204, and/or the image recognition data 209 associated with the user 102 (e.g., previously data generated 200, 202, 204, 209). Additionally or alternatively, the training data 230 can include the population profile data 212 for users with similar properties (e.g., physical properties) as the user 102.

The neural network trainer 226 trains the neural network implemented by the neural network processor 224 using the training data 230 to identify reference or optimal ergonomic forms for performing movement(s) that are based on or directed to properties of the user 102. One or more avatar position models 234 are generated as a result of the neural network training. The avatar position model(s) 234 define position(s), posture(s), ranges(s) of motion, speed(s), muscle tension level(s), etc. to be demonstrated by the avatar or digital coach with respect to movement(s) associated with a task (e.g., lifting a box) to promote and/or protect musculoskeletal integrity. The avatar position model(s) 234 are stored in a database 236. The databases 232, 236 may be the same storage device or different storage devices.

The avatar position analyzer 220 executes the avatar position model(s) 234 to generate instructions for controlling an avatar with respect to movement(s) that represent reference or optimal ergonomic form(s) for the user 102 when performing the movement(s). The avatar position model(s) 234 executed by the avatar position analyzer 220 can be selected based on a task to be performed by the user 102 (e.g., as specified in the user profile data 210). The instructions for controlling movement(s) and/or form(s) to be illustrated by the avatar are stored as avatar control instruction(s) 236 in the database 206.

The avatar generator 216 implements the avatar control instruction(s) 236 to cause the avatar to perform the movements with the ergonomic form(s) specified in the instruction(s) 236. For example, the avatar generator 216 can cause the avatar to bend its knees to demonstrate optimal posture for lifting a box based on the instruction(s) 236. The avatar generator 216 communicates with one or more of the processor 108 (e.g., in examples where the XR coach controller 120 is implemented by a different processor) and/or the display controller 110 of the XR device 104 to cause the avatar defined by the avatar property data 218 and illustrating the ergonomic form(s) defined by the instruction(s) 236 to be output by the XR device 104.

In the example of FIGS. 1 and 2, the sensor(s) 112, 116, 118 generate the sensor data 200, 202, 204 associated with the user 102 as the user 102 interacts with the avatar or digital coach generated by the avatar generator 216 and output by the XR device 104. The example XR coach controller 120 of FIG. 2 includes a feedback generator 238. In the example of FIG. 2, the feedback generator 238 analyzes the user position data 200, the strain sensor data 202, the image data 204, and/or the image recognition data 209 to compare the form(s) of the user 102 to the reference or optimal ergonomic form(s) demonstrated by the avatar. In some examples, the feedback generator 238 determines if the user form(s) (e.g., position(s), range(s) of motion(s), speed(s), muscle tension, etc. of the body part(s) of the user 102) align or substantially align with the corresponding reference or optimal ergonomic form(s) (e.g., position(s), range(s) of motion(s), speed(s), muscle tension, etc., demonstrated by the avatar).

In some examples, the feedback generator 238 analyzes the user position data 200, the strain sensor data 202, the image data 204, and/or the image recognition data 209 to determine position(s) of one or more portion(s) of the body of the user. For example, the feedback generator 238 can determine an angle at which an arm of the user 102 is disposed when the arm is raised above the user's head based on locations of joints (e.g., keypoints representative of the shoulder joint, the elbow joint, the wrist joint) detected in the image data 204 and stored as the image recognition data 209. In some examples, the feedback generator 238 analyzes image data collected by the image sensor(s) 118 from multiple views to determine position(s) of the body parts of the user 102. In other examples, the feedback generator 238 can analyze the user position data 200 to determine locations of the body part(s) of the user 102 relative to a reference location via, for instance, motion capture analysis.

The feedback generator 238 compares the form(s) of the body part(s) of the user 102 to the reference or optimal ergonomic form(s) of the corresponding portion(s) of the avatar as determined by the neural network analysis and defined by the avatar control instructions 236. For example, the feedback generator 238 can map the position(s) of the body part(s) of the user 102 to the reference (e.g., optimal) position(s) of the corresponding body part(s) represented by the avatar. The feedback generator 238 determines if the position(s) of the body part(s) of the user 102 align or substantially align with the reference position(s) demonstrated by the avatar that correspond to proper ergonomic form. Additionally or alternatively, the feedback generator 238 can compare the position(s) of the body part(s) of the user 102 to the reference position(s) or movement(s) defined in the task reference data 214.

The feedback generator 238 determines if the form(s) of the body part(s) of the user 102 align or substantially align with the corresponding ergonomic form(s) of the avatar based on alignment threshold rule(s) 240. The alignment threshold rule(s) can define, for example, an allowable threshold (e.g., percentage) of a difference between a position of a body part of the user 102 and the reference position such that the form of the user satisfies the reference ergonomic form. The alignment threshold rule(s) 240 can be defined by user input(s).

In other examples, the feedback generator 238 can analyze speed(s) at which the user 102 is performing a movement, muscle tension exerted, etc. based on the sensor data 200, 202, 204 to determine differences between the efforts exerted by the user 102 in connection with the movement(s) and the recommended speeds, muscle tension, etc. demonstrated by the avatar. The alignment threshold rule(s) 240 can define corresponding thresholds for comparison.

In the example of FIG. 2, the feedback generator 238 generates instruction(s) to cause feedback with respect to the movement(s) and associated form(s) of the user relative to the movement(s) and associated reference or optimal ergonomic form(s) demonstrated by the avatar to be presented via the XR device 104. In some examples, the feedback generator 238 generates instructions to cause the display controller 110 of the XR device 104 to change a graphical feature (e.g., color, an appearance) of the avatar based on whether or not the form(s) (e.g., body part position(s)) of the user 102 are aligned or substantially aligned with the form(s) of the avatar. For example, the feedback generator 238 can generate instructions for the avatar to be displayed as green when feedback generator 238 determines that the posture of the user 102 satisfy the alignment threshold rule(s) 240 and, thus, is aligned or substantially aligned with the posture of the avatar. The feedback generator 238 can generate instructions for the avatar to be displayed as red when feedback generator 238 determines that the posture of the user 102 does not satisfy the alignment threshold rule(s) 240 and, thus, is not aligned or not substantially aligned with the posture of the avatar. The feedback generator 238 transmits the instructions to the display controller of the XR device 104.

In some examples, the feedback generator 238 generates graphical representation(s) 242 of the user 102 in particular position(s) based on the analysis of the sensor data 200, 202, 204. The feedback generator 238 instructs the display controller 110 of the XR device 104 to present the graphical representation 242 of the user 102 with the avatar. For example, the graphical representation 242 of the user can be illustrated as overlaying the avatar in a corresponding position. The overlaying of the graphical representation 242 and the avatar can provide graphical indications of differences between the form of the user 102 and the reference or optimal ergonomic form demonstrated by the avatar.

In some examples, the feedback generator 238 instructs the display controller 110 of the XR device 104 to output the graphical representations 242 showing positions of the user 102 relative to the avatar over time. Such information can inform the user 102 as to whether his or her form is improving over time with respect to ergonomic performance of the movement(s). In some examples, the feedback generator 238 can instruct the display controller to output image data showing positions of other users relative to the avatar over time based on the population profile data 212 to show the user 102 how the user 102 compares to other users.

Additionally or alternatively, the feedback generator 238 can instruct the XR device 104 and/or the other user device(s) 114 to provide other types of feedback with respect to ergonomic performance of the user 102. For example, the feedback generator 238 can generate audio output(s) informing the user 102 of whether or not he or she is performing the movement correctly (e.g., based on the comparison of the sensor data 200, 202, 204 and/or data 209 derived therefrom to the alignment threshold rule(s) 240) and/or textual instructions as to how to perform a movement for display.

Additionally or alternatively, the feedback generator 238 can generate instructions to provide haptic feedback to the user 102 via the haptic feedback actuator(s) 124. In some examples, the feedback generator 238 generates instructions for haptic feedback to be provided based on the analysis of the movement(s) of the user 102 relative to the avatar to alert the user 102 with respect to, for instance, improper form. In other examples, the feedback generator 238 generates instructions for haptic feedback independent of the presentation of the avatar to serve as a reminder to the user 102 to be alert to as to ergonomic form when performing movement(s). For instance, the feedback generator 238 can instruct the haptic feedback actuator(s) 124 to generate haptic feedback in response to detection of a change in a position of a body part of the user based on analysis of the sensor data 200, 202, 204.

In some examples, the feedback generator 238 transmits data regarding the performance of the user 102 relative to the avatar to user device(s) 114 associated with authorized third parties to enable the third parties to analyze the performance of the user 102 over time. The third party can include, for example, a medical professional. In some examples, the feedback generator 238 transmits the data collected by the sensor(s) 200, 202, 204, the image recognition data 209, and/or the graphical representation(s) 242 of the user 102 overlaying the avatar for display at the output device(s) 114. Thus, the authorized third party can track changes in performance of movement(s) by the user 102 over time.

In the example of FIG. 2, the user position data 200, the strain sensor data 202, and/or the image data 204, the image recognition data 209, and/or the graphical representation(s) 242 can be used to refine the avatar position model(s) 234 as part of feedback training. For example, changes in force exerted by the user 102 as defined in the strain sensor data 202 collected over time can be used to adjust the instructions generated by the avatar position analyzer 220 with respect to, for instance, a duration of time for which a position is held by the avatar and, thus, the user 102. Similarly, changes in positions of the body part(s) of the user 102 over time can indicate injuries. In other examples, the graphical representation(s) 242 of the user 102 performing the movement(s) or form(s) demonstrated by the avatar can be used for feedback training. The avatar position model(s) 234 can be updated to account for performance by the user 102 and to provide updated recommendations to the user 102 based on updates to the sensor data 200, 202, 204 and/or the user profile data 210 over time (e.g., indicating a change in a health condition of the user 102, etc.).

While an example manner of implementing the XR coach controller 120 of FIG. 1 is illustrated in FIG. 2, one or more of the elements, processes and/or devices illustrated in FIG. 2 may be combined, divided, re-arranged, omitted, eliminated and/or implemented in any other way. Further, the example database 206, the example image data analyzer 207, the example signal modifier 208, the example avatar generator 216, the example avatar position analyzer 220, the example feedback generator 238 and/or, more generally, the example XR coach controller 120 of FIG. 2 may be implemented by hardware, software, firmware and/or any combination of hardware, software and/or firmware. Thus, for example, any of the example database 206, the example image data analyzer 207, the example signal modifier 208, the example avatar generator 216, the example avatar position analyzer 220, the example feedback generator 238 and/or, more generally, the example XR coach controller 120 could be implemented by one or more analog or digital circuit(s), logic circuits, programmable processor(s), programmable controller(s), graphics processing unit(s) (GPU(s)), digital signal processor(s) (DSP(s)), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example database 206, the example image data analyzer 207, the example signal modifier 208, the example avatar generator 216, the example avatar position analyzer 220, and/or the example feedback generator 238 is/are hereby expressly defined to include a non-transitory computer readable storage device or storage disk such as a memory, a digital versatile disk (DVD), a compact disk (CD), a Blu-ray disk, etc. including the software and/or firmware. Further still, the example XR coach controller 120 may include one or more elements, processes and/or devices in addition to, or instead of, those illustrated in FIG. 2, and/or may include more than one of any or all of the illustrated elements, processes, and devices. As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

While an example manner of implementing the first computing system 222 is illustrated in FIG. 2, one or more of the elements, processes, and/or devices illustrated in FIG. 2 may be combined, divided, re-arranged, omitted, eliminated and/or implemented in any other way. Further, the example neural network processor 224, the example trainer 226, the example training controller 228, the example database(s) 232, 236 and/or, more generally, the example first computing system 222 of FIG. 2 may be implemented by hardware, software, firmware and/or any combination of hardware, software and/or firmware. Thus, for example, any of the example neural network processor 224, the example trainer 226, the example training controller 228, the example database(s) 232, 236 and/or, more generally, the example first computing system 222 could be implemented by one or more analog or digital circuit(s), logic circuits, programmable processor(s), programmable controller(s), graphics processing unit(s) (GPU(s)), digital signal processor(s) (DSP(s)), application specific integrated circuit(s) (ASIC(s)), programmable logic device(s) (PLD(s)) and/or field programmable logic device(s) (FPLD(s)). When reading any of the apparatus or system claims of this patent to cover a purely software and/or firmware implementation, at least one of the example neural network processor 224, the example trainer 226, the example training controller 228, and/or the example database(s) 232, 236 is/are hereby expressly defined to include a non-transitory computer readable storage device or storage disk such as a memory, a digital versatile disk (DVD), a compact disk (CD), a Blu-ray disk, etc. including the software and/or firmware. Further still, the example first computing system 222 may include one or more elements, processes, and/or devices in addition to, or instead of, those illustrated in FIG. 2, and/or may include more than one of any or all of the illustrated elements, processes, and devices. As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

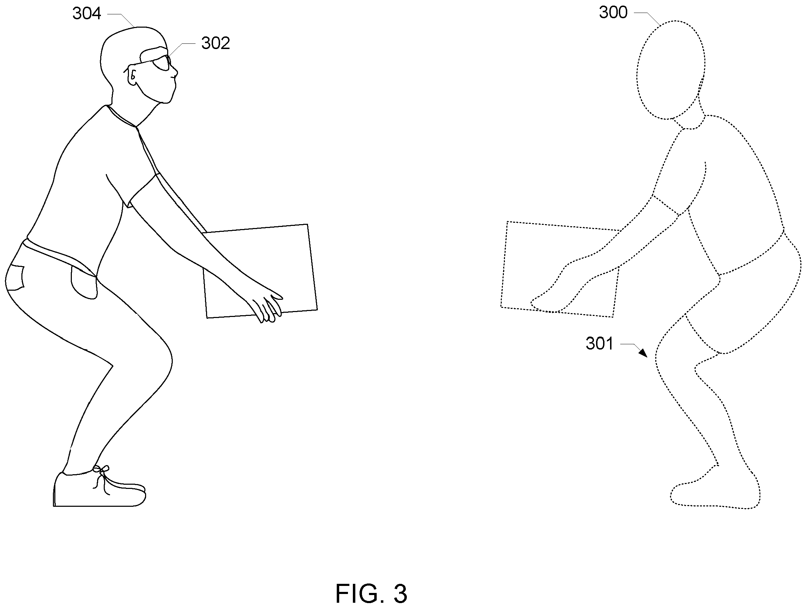

FIG. 3 illustrates an example avatar 300 generated by the example extended reality (XR) coach controller 120 of FIGS. 1 and/or 2 and presented via an XR device 302 (e.g., the XR device 104 of FIGS. 1 and 2) worn by a user 304. In the example of FIG. 3, the XR device 302 includes glasses, such as augmented reality glasses.

As shown in FIG. 3, the example avatar 300 demonstrates reference (e.g., optimal) ergonomic form 301 for performing a task including lifting a box. For instance, in FIG. 3, the avatar 300 is shown with its knees bent and back straight or substantially straight. The ergonomic form 301 of the avatar 300 can be determined by the avatar position analyzer 220 based on execution of the neural network-based avatar position model(s) 234 and properties of the user 304 (e.g., the user profile data 210) and/or similar users as the user 304 (e.g., the population profile data 212). The avatar 300 can demonstrate other movements and associated ergonomic forms in connection with lifting the box, such as standing upright with the box, walking with the box, etc.

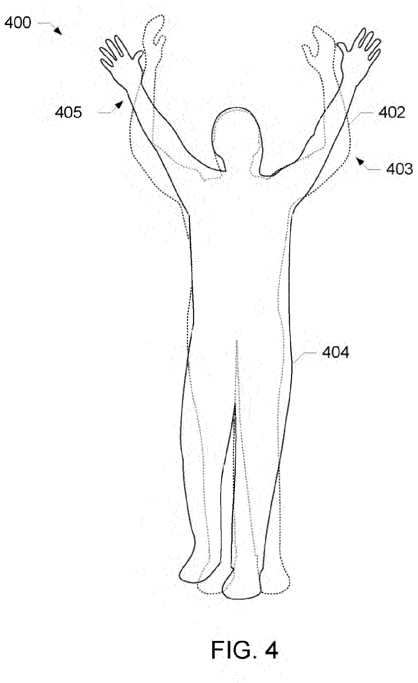

FIG. 4 illustrates an example graphical user-avatar overlay 400 generated by the example XR coach controller 120 of FIGS. 1 and/or 2. The example graphical user-avatar overlay 400 can be presented via the XR device 104, 302 to provide a user with graphical feedback regarding his or her form relative to the ergonomic form demonstrated by an avatar or digital coach 402 generated by the XR coach controller 120.

In the example of FIG. 4, the avatar 402 demonstrates a first ergonomic form 403 (e.g., a reference ergonomic form, a first ergonomic position). For instance, the first ergonomic form 403 shown in FIG. 4 represents a recommended form for a user to raise his or her arms above the head to retrieve an object (e.g., by slightly bending the arms to relieve stress on the elbow and/or shoulder joints and keeping both feet on the ground). The graphical user-avatar overlay 400 of FIG. 4 includes a graphical representation 404 (e.g., the graphical representation(s) 242) of a user, such as the user 102, 304 of FIGS. 1 and 3 in a second position or a second form 405. For instance, the second form 405 shows that the user's arms are straight or substantially straight and one foot is lifted relative to the other foot. As shown in FIG. 4, the graphical representation 404 of the user in the second form 405 is presented as overlaying the image of the avatar 402 in the first ergonomic form 403. Thus, any differences between the form 405 of the user as represented by the graphical representation 404 and the form 403 of the avatar are visible to the user.

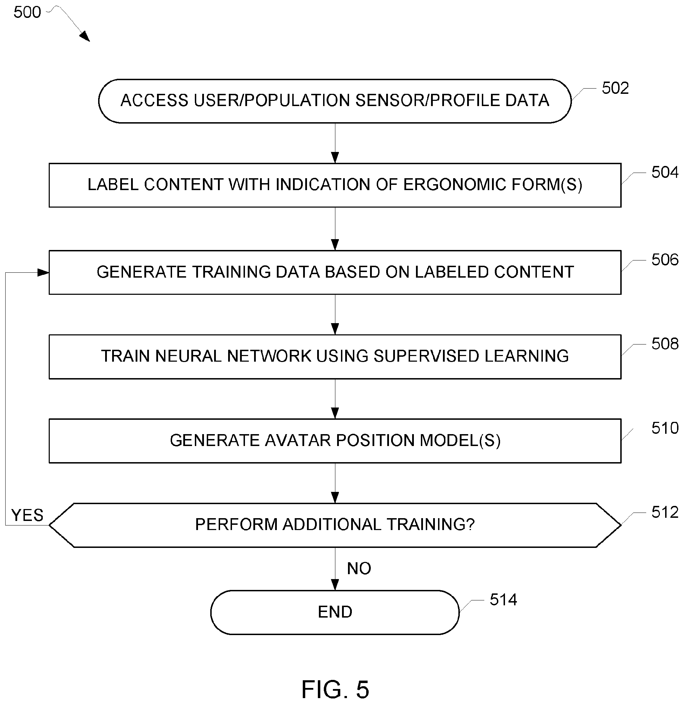

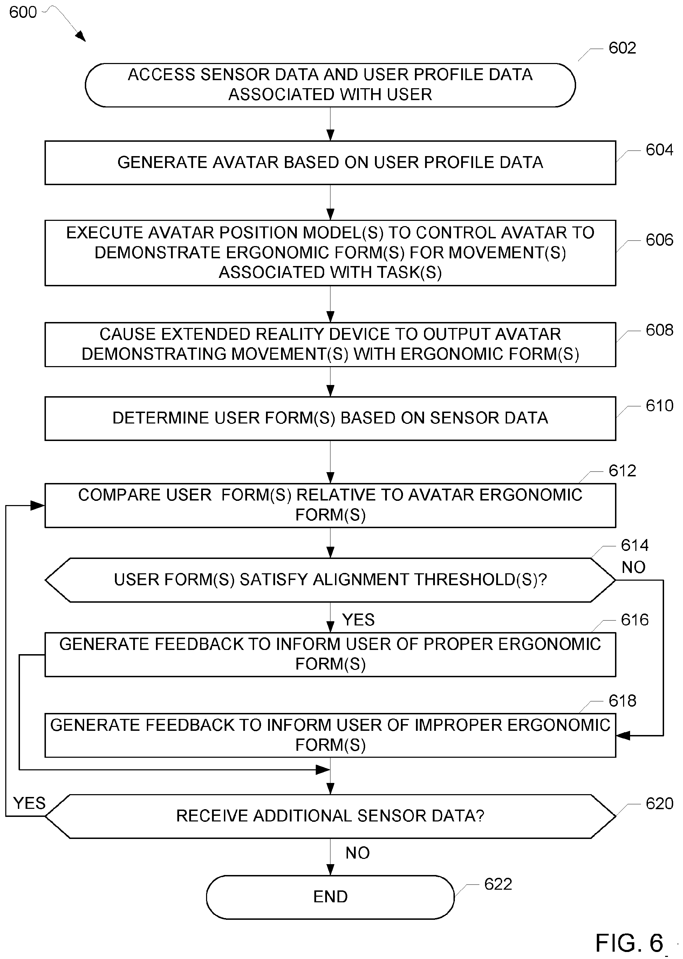

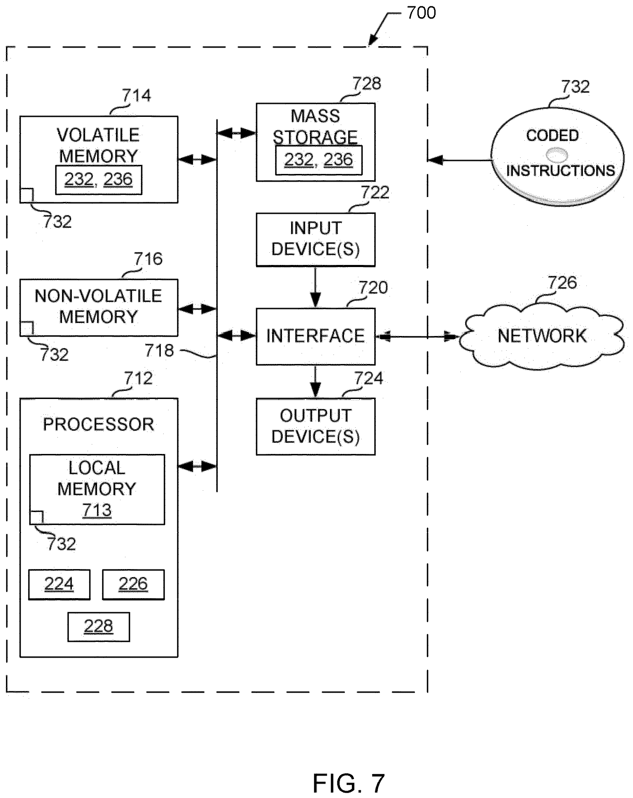

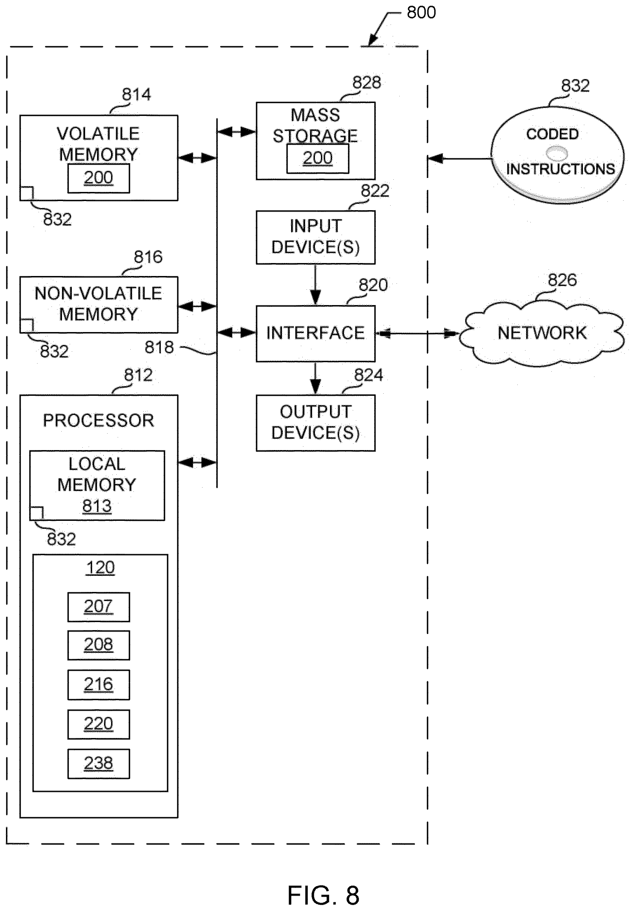

A flowchart representative of example hardware logic, machine readable instructions, hardware implemented state machines, and/or any combination thereof for implementing the example first computing system 222 is shown in FIG. 5. A flowchart representative of example hardware logic, machine readable instructions, hardware implemented state machines, and/or any combination thereof for implementing the example XR coach controller 120 is shown in FIG. 6. The machine readable instructions may be one or more executable programs or portion(s) of an executable program for execution by a computer processor and/or processor circuitry, such as the processor 712, 812 shown in the example processor platforms 700, 800 discussed below in connection with FIGS. 7 and 8. The program(s) may be embodied in software stored on a non-transitory computer readable storage medium such as a CD-ROM, a floppy disk, a hard drive, a DVD, a Blu-ray disk, or a memory associated with the processor(s) 712, 812, but the entire program and/or parts thereof could alternatively be executed by a device other than the processor(s) 712, 812 and/or embodied in firmware or dedicated hardware. Further, although the example program(s) are described with reference to the flowchart(s) illustrated in FIGS. 5 and 6, many other methods of implementing the example first computing system 222 and/or the example XR coach controller 120 may alternatively be used. For example, the order of execution of the blocks may be changed, and/or some of the blocks described may be changed, eliminated, or combined. Additionally or alternatively, any or all of the blocks may be implemented by one or more hardware circuits (e.g., discrete and/or integrated analog and/or digital circuitry, an FPGA, an ASIC, a comparator, an operational-amplifier (op-amp), a logic circuit, etc.) structured to perform the corresponding operation without executing software or firmware. The processor circuitry may be distributed in different network locations and/or local to one or more devices (e.g., a multi-core processor in a single machine, multiple processors distributed across a server rack, etc.).

The machine readable instructions described herein may be stored in one or more of a compressed format, an encrypted format, a fragmented format, a compiled format, an executable format, a packaged format, etc. Machine readable instructions as described herein may be stored as data or a data structure (e.g., portions of instructions, code, representations of code, etc.) that may be utilized to create, manufacture, and/or produce machine executable instructions. For example, the machine readable instructions may be fragmented and stored on one or more storage devices and/or computing devices (e.g., servers) located at the same or different locations of a network or collection of networks (e.g., in the cloud, in edge devices, etc.). The machine readable instructions may require one or more of installation, modification, adaptation, updating, combining, supplementing, configuring, decryption, decompression, unpacking, distribution, reassignment, compilation, etc. in order to make them directly readable, interpretable, and/or executable by a computing device and/or other machine. For example, the machine readable instructions may be stored in multiple parts, which are individually compressed, encrypted, and stored on separate computing devices, wherein the parts when decrypted, decompressed, and combined form a set of executable instructions that implement one or more functions that may together form a program such as that described herein.

In other examples, the machine readable instructions may be stored in a state in which they may be read by processor circuitry, but require addition of a library (e.g., a dynamic link library (DLL)), a software development kit (SDK), an application programming interface (API), etc. in order to execute the instructions on a particular computing device or other device. In other examples, the machine readable instructions may need to be configured (e.g., settings stored, data input, network addresses recorded, etc.) before the machine readable instructions and/or the corresponding program(s) can be executed in whole or in part. Thus, machine readable media, as used herein, may include machine readable instructions and/or program(s) regardless of the particular format or state of the machine readable instructions and/or program(s) when stored or otherwise at rest or in transit.

The machine readable instructions described herein can be represented by any past, present, or future instruction language, scripting language, programming language, etc. For example, the machine readable instructions may be represented using any of the following languages: C, C++, Java, C#, Perl, Python, JavaScript, HyperText Markup Language (HTML), Structured Query Language (SQL), Swift, etc.

As mentioned above, the example processes of FIGS. 5 and 6 may be implemented using executable instructions (e.g., computer and/or machine readable instructions) stored on a non-transitory computer and/or machine readable medium such as a hard disk drive, a flash memory, a read-only memory, a compact disk, a digital versatile disk, a cache, a random-access memory and/or any other storage device or storage disk in which information is stored for any duration (e.g., for extended time periods, permanently, for brief instances, for temporarily buffering, and/or for caching of the information). As used herein, the term non-transitory computer readable medium is expressly defined to include any type of computer readable storage device and/or storage disk and to exclude propagating signals and to exclude transmission media.

"Including" and "comprising" (and all forms and tenses thereof) are used herein to be open ended terms. Thus, whenever a claim employs any form of "include" or "comprise" (e.g., comprises, includes, comprising, including, having, etc.) as a preamble or within a claim recitation of any kind, it is to be understood that additional elements, terms, etc. may be present without falling outside the scope of the corresponding claim or recitation. As used herein, when the phrase "at least" is used as the transition term in, for example, a preamble of a claim, it is open-ended in the same manner as the term "comprising" and "including" are open ended. The term "and/or" when used, for example, in a form such as A, B, and/or C refers to any combination or subset of A, B, C such as (1) A alone, (2) B alone, (3) C alone, (4) A with B, (5) A with C, (6) B with C, and (7) A with B and with C. As used herein in the context of describing structures, components, items, objects and/or things, the phrase "at least one of A and B" is intended to refer to implementations including any of (1) at least one A, (2) at least one B, and (3) at least one A and at least one B. Similarly, as used herein in the context of describing structures, components, items, objects and/or things, the phrase "at least one of A or B" is intended to refer to implementations including any of (1) at least one A, (2) at least one B, and (3) at least one A and at least one B. As used herein in the context of describing the performance or execution of processes, instructions, actions, activities and/or steps, the phrase "at least one of A and B" is intended to refer to implementations including any of (1) at least one A, (2) at least one B, and (3) at least one A and at least one B. Similarly, as used herein in the context of describing the performance or execution of processes, instructions, actions, activities and/or steps, the phrase "at least one of A or B" is intended to refer to implementations including any of (1) at least one A, (2) at least one B, and (3) at least one A and at least one B.

As used herein, singular references (e.g., "a", "an", "first", "second", etc.) do not exclude a plurality. The term "a" or "an" entity, as used herein, refers to one or more of that entity. The terms "a" (or "an"), "one or more", and "at least one" can be used interchangeably herein. Furthermore, although individually listed, a plurality of means, elements or method actions may be implemented by, e.g., a single unit or processor. Additionally, although individual features may be included in different examples or claims, these may possibly be combined, and the inclusion in different examples or claims does not imply that a combination of features is not feasible and/or advantageous.

FIG. 5 is a flowchart representative of example machine readable instructions 500 that, when executed by the example first computing system 222 of FIG. 2, cause the first computing system 222 to train a neural network to determine ergonomic form(s) (e.g. position(s), orientation(s), posture(s), ranges(s) of motion, speed(s), muscle tension level(s), etc.) to be demonstrated by an avatar or digital coach with respect to movement(s) associated with a task (e.g., lifting a box). The example instructions 500 of FIG. 5, when executed by the first computing system 222 of FIG. 2, result in a neural network and/or a model thereof, that can be distributed to other computing systems, such as the avatar position analyzer 220 of the example XR coach controller 120 of FIG. 2.

The example instructions 500 begin with the training controller 228 accessing sensor data and/or profile data associated with user(s) and/or population(s) stored in the database 232 (block 502). The sensor data can include, for example, previously generated user position data 200, strain sensor data 202, image data 204, user profile data 210, population profile data 212, task reference data 214. In some examples, the data includes the graphical representation(s) 242 of the user generated by the feedback generator 238 as part of feedback training. In some examples, the sensor data is associated with a particular portion of the body of interest with respect to strain events, such as a shoulder, a knee, a wrist, neck, back, etc.

The example training controller 228 labels the data as with respect to ergonomic form(s) (block 504). For example, when the sensor data includes image data of a user performing a movement, the training controller 228 labels the image(s) corresponding to the user in a position that corresponds to reference (e.g., optimal, proper) ergonomic form with respect to one or more body parts of the user. In other examples, the training controller 228 labels muscle strain data with thresholds for detecting muscle tension level(s) exerted by user(s) in certain position(s). In some examples, the data is labeled for a particular user (e.g., the user 102 of FIG. 1) based on properties of the user such as an age, gender, height, etc. Also, the data can be labeled for specific tasks and/or portions of the body.

The example training controller 228 generates the training data 230 based on the labeled sensor data (block 506).

The example training controller 228 instructs the neural network trainer 226 to perform training of the neural network 224 using the training data 230 (block 508). In the example of FIG. 5, the training is based on supervised learning. As a result of the training, the avatar position model(s) 234 are generated (block 510). Based on the avatar position model(s) 234, the neural network is trained to identify ergonomic form(s) (e.g., posture(s), position(s), orientation(s), range(s) of motion, speed, muscle tension level(s), etc.) with respect to movement(s) of an avatar or digital coach that represent reference (e.g., optimal) ergonomic form(s) for the user. The avatar position model(s) 234 can be stored in the database 236 for access by the avatar position analyzer 220 of the XR coach controller 120 of FIG. 2. The example instructions 500 of FIG. 5 end when no additional training (e.g., retraining) is to be performed (blocks 512, 514).

FIG. 6 is a flowchart representative of example machine readable instructions 600 that, when executed by the XR coach controller 120 of FIGS. 1 and/or 2, cause the XR coach controller 120 to generate an extended reality avatar that demonstrates reference or optimal ergonomic form(s) for a user (e.g., the user 102) when performing movement(s) associated with task(s). The example instructions 600 can be executed by one or more processor(s) of user device(s) such as the XR device 104 of FIG. 1 and/or cloud-based device(s).

The example instructions 600 begin with the XR coach controller 120 accessing sensor data and user profile data 210 associated with a user (e.g., the user 102 of FIG. 1). The sensor data can include the user position data 200, the strain sensor data 202, and/or the image data 204 (block 502).

The avatar generator 216 of FIG. 2 generates an avatar based on the user profile data 210 (block 504). The avatar position analyzer 220 executes the avatar position model(s) 234 to determine ergonomic form(s) 301, 403 (e.g., posture(s), position(s), orientation(s), range(s) of motion, speed, muscle tension level(s), etc.) with respect to movement(s) of an avatar or digital coach for particular task(s) that represent reference (e.g., optimal) ergonomic form(s) for the user (block 606). The avatar generator 216 causes the XR device 104 to output the avatar for presentation, where the avatar demonstrates the reference (e.g., optimal) ergonomic form(s) 301, 403 for performing the movement(s) associated with the task(s) (block 608).

The feedback generator 238 of FIG. 2 analyzes the user position data 200, the strain sensor data 202, the image data 204, and/or the image recognition data 209 to determine form(s) (e.g., position(s), orientation(s), range(s) of motion, speed(s), muscle tension level(s)) associated with movement(s) by the user 102 (e.g., when performing a task) (block 610). For example, the feedback generator 238 can determine a position of a user's arm based on joint keypoint analysis from the image data 204 and/or the image recognition data 209. In some examples, the feedback generator 238 can detect weight transfer(s) between portion(s) of the user's body based on the user position data 200 (e.g., weight shift between legs and/or feet). In some examples, the sensor data 200, 202, 204 is collected during presentation of the avatar via the XR device 104.

The feedback generator 238 compares the form(s) 405 of the user 102 to the ergonomic form(s) 301, 403 demonstrated by the avatar (block 612). The feedback generator 238 determines if the user form(s) 405 are aligned or substantially aligned with the ergonomic form(s) 301, 403 demonstrated by the avatar within threshold amount(s) defined by the alignment threshold rule(s) 240 (block 614).

The feedback generator 238 generates feedback to be output to the user via the XR device 104 and/or the other user device(s) 114 in response to the analysis of the form(s) of the user relative to the ergonomic form(s) presented by the avatar (blocks 616, 618). The feedback can include graphical representations 242 of the user that overlay the image(s) of the avatar. The feedback provides indications of whether the user is executing proper or improper ergonomic form(s). For example, a color of the avatar can change based on whether the feedback generator 238 determines that the form(s) of the user are aligned or substantially aligned with the ergonomic form(s) of the avatar. In addition to or as an alternative to visual feedback, the feedback generated by the feedback generator 238 can include audio output(s) and/or haptic feedback.

The XR coach controller 120 continues to analyze the user's ergonomic form(s) 405 relative to the ergonomic form(s) 301, 403 demonstrated by the avatar as additional sensor data 200, 202, 204 is received by the XR coach controller 120 (block 620). The example instructions 600 of FIG. 6 end when no further sensor data 200, 202, 204 is received (block 622).

FIG. 7 is a block diagram of an example processor platform 700 structured to execute the instructions of FIG. 5 to implement the first computing system 222 of FIG. 2. The processor platform 700 can be, for example, a server, a personal computer, a workstation, a self-learning machine (e.g., a neural network), an Internet appliance, or any other type of computing device.

The processor platform 700 of the illustrated example includes a processor 712. The processor 712 of the illustrated example is hardware. For example, the processor 712 can be implemented by one or more integrated circuits, logic circuits, microprocessors, GPUs, DSPs, or controllers from any desired family or manufacturer. The hardware processor may be a semiconductor based (e.g., silicon based) device. In this and other examples, the processor implements the example neural network processor 224, the example trainer 226, and the example training controller 228.

The processor 712 of the illustrated example includes a local memory 713 (e.g., a cache). The processor 712 of the illustrated example is in communication with a main memory including a volatile memory 714 and a non-volatile memory 716 via a bus 718. The volatile memory 714 may be implemented by Synchronous Dynamic Random Access Memory (SDRAM), Dynamic Random Access Memory (DRAM), RAMBUS^{®} Dynamic Random Access Memory (RDRAM^{®}) and/or any other type of random access memory device. The non-volatile memory 716 may be implemented by flash memory and/or any other desired type of memory device. Access to the main memory 714, 716 is controlled by a memory controller.

The processor platform 700 of some examples also includes an interface circuit 720. The interface circuit 720 may be implemented by any type of interface standard, such as an Ethernet interface, a universal serial bus (USB), a Bluetooth^{®} interface, a near field communication (NFC) interface, and/or a PCI express interface.

In some examples, one or more input devices 722 are connected to the interface circuit 720. The input device(s) 722 permit(s) a user to enter data and/or commands into the processor 712. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 724 are also connected to the interface circuit 720 of some examples including the illustrated example. The output devices 724 can be implemented, for example, by display devices (e.g., a light emitting diode (LED), an organic light emitting diode (OLED), a liquid crystal display (LCD), a cathode ray tube display (CRT), an in-place switching (IPS) display, a touchscreen, etc.), a tactile output device, a printer and/or speaker. The interface circuit 720 of some examples including the illustrated example, thus, typically includes a graphics driver card, a graphics driver chip and/or a graphics driver processor.

The interface circuit 720 of some examples including the illustrated example also includes a communication device such as a transmitter, a receiver, a transceiver, a modem, a residential gateway, a wireless access point, and/or a network interface to facilitate exchange of data with external machines (e.g., computing devices of any kind) via a network 726. The communication can be via, for example, an Ethernet connection, a digital subscriber line (DSL) connection, a telephone line connection, a coaxial cable system, a satellite system, a line-of-site wireless system, a cellular telephone system, etc.

The processor platform 700 of some examples including the illustrated example also includes one or more mass storage devices 728 for storing software and/or data. Examples of such mass storage devices 728 include floppy disk drives, hard drive disks, compact disk drives, Blu-ray disk drives, redundant array of independent disks (RAID) systems, and digital versatile disk (DVD) drives.

Coded instructions 732 of FIG. 7 may be stored in the mass storage device 728, in the volatile memory 714, in the non-volatile memory 716, and/or on a removable non-transitory computer readable storage medium such as a CD or DVD.

FIG. 8 is a block diagram of an example processor platform 800 structured to execute the instructions of FIG. 6 to implement the example extended reality coach controller 120 of FIGS. 1 and/or 2. The processor platform 800 can be, for example, a server, a personal computer, a workstation, a self-learning machine (e.g., a neural network), a mobile device (e.g., a cell phone, a smart phone, a tablet such as an iPad^{™}), a personal digital assistant (PDA), an Internet appliance, a headset or other wearable device, or any other type of computing device.

The processor platform 800 of some examples including the illustrated example includes a processor 812. The processor 812 of some examples including the illustrated example is hardware. For example, the processor 812 can be implemented by one or more integrated circuits, logic circuits, microprocessors, GPUs, DSPs, or controllers from any desired family or manufacturer. The hardware processor may be a semiconductor based (e.g., silicon based) device. In this and other examples, the processor implements the example signal modifier 208, example image data analyzer 207, the example avatar generator 216, the example avatar position analyzer 220, and the example feedback generator 238.

The processor 812 of some examples including the illustrated example includes a local memory 813 (e.g., a cache). The processor 812 of some examples including the illustrated example is in communication with a main memory including a volatile memory 814 and a non-volatile memory 816 via a bus 818. The volatile memory 814 may be implemented by Synchronous Dynamic Random Access Memory (SDRAM), Dynamic Random Access Memory (DRAM), RAMBUS^{®} Dynamic Random Access Memory (RDRAM^{®}) and/or any other type of random access memory device. The non-volatile memory 816 may be implemented by flash memory and/or any other desired type of memory device. Access to the main memory 814, 816 is controlled by a memory controller.

The processor platform 800 of some examples including the illustrated example also includes an interface circuit 820. The interface circuit 820 may be implemented by any type of interface standard, such as an Ethernet interface, a universal serial bus (USB), a Bluetooth^{®} interface, a near field communication (NFC) interface, and/or a PCI express interface.

In some examples including the illustrated example, one or more input devices 822 are connected to the interface circuit 820. The input device(s) 822 permit(s) a user to enter data and/or commands into the processor 812. The input device(s) can be implemented by, for example, an audio sensor, a microphone, a camera (still or video), a keyboard, a button, a mouse, a touchscreen, a track-pad, a trackball, isopoint and/or a voice recognition system.

One or more output devices 824 are also connected to the interface circuit 820 of some examples including the illustrated example. The output devices 824 can be implemented, for example, by display devices (e.g., a light emitting diode (LED), an organic light emitting diode (OLED), a liquid crystal display (LCD), a cathode ray tube display (CRT), an in-place switching (IPS) display, a touchscreen, etc.), a tactile output device, a printer and/or speaker. The interface circuit 820 of some examples including the illustrated example, thus, typically includes a graphics driver card, a graphics driver chip and/or a graphics driver processor.

The interface circuit 820 of some examples including the illustrated example also includes a communication device such as a transmitter, a receiver, a transceiver, a modem, a residential gateway, a wireless access point, and/or a network interface to facilitate exchange of data with external machines (e.g., computing devices of any kind) via a network 826. The communication can be via, for example, an Ethernet connection, a digital subscriber line (DSL) connection, a telephone line connection, a coaxial cable system, a satellite system, a line-of-site wireless system, a cellular telephone system, etc.

The processor platform 800 of the illustrated example also includes one or more mass storage devices 828 for storing software and/or data. Examples of such mass storage devices 828 include floppy disk drives, hard drive disks, compact disk drives, Blu-ray disk drives, redundant array of independent disks (RAID) systems, and digital versatile disk (DVD) drives.

Coded instructions 832 of FIG. 8 may be stored in the mass storage device 828, in the volatile memory 814, in the non-volatile memory 816, and/or on a removable non-transitory computer readable storage medium such as a CD or DVD.

From the foregoing, it will be appreciated that example systems, methods, apparatus, and articles of manufacture have been disclosed that generate an avatar or digital coach that demonstrates reference or optimal ergonomic form for performing movement(s) associated with a task. Examples disclosed herein use extended reality (e.g., augmented reality, mixed reality) to inform a user as to how to perform the task to promote and/or preserve musculoskeletal integrity of to body part(s) (e.g., shoulder, back, legs) of the user. Examples disclosed herein generate an avatar based on properties of the user such as gender, height, etc. Examples disclosed herein perform neural network analysis to determine reference or optimal ergonomic form(s) (e.g., position(s), posture(s), etc.) with respect to movement(s) to be performed by the user in connection with a task and based on the properties of the user and/or similar users. In some examples disclosed herein, the avatar is presented via an extended reality device (e.g., augmented reality glasses) and demonstrates how to perform the movement(s) with the ergonomic form(s) determined via the neural network analysis. Some examples disclosed herein provide the user with feedback, such as a graphical representation of the user shown with (e.g., overlaying) the avatar, to inform the user as to the quality of his or her form relative to the optimal ergonomic forms illustrated by the avatar.

Example extended reality systems, apparatus, and methods for musculoskeletal ergonomic improvement are disclosed herein. Further examples and combinations thereof include the following
Clause 1 includes an apparatus including an avatar generator to generate an avatar based one or more properties of a user; an avatar position analyzer to determine a first ergonomic form for a movement based on the one or more properties of the user, the avatar generator to cause an output device to display the avatar in the first ergonomic form; and a feedback generator to determine a second form associated with movement of the user based on sensor data collected via one or more sensors associated with the user; generate a graphical representation of the user in the second form; and cause the output device to display the graphical representation of the user with the avatar.
Clause 2 includes the apparatus of clause 1, wherein the sensor data includes position data for one or more body parts of the user.
Clause 3 includes the apparatus of clauses 1 or 2, wherein the sensor data includes image data including the user.
Clause 4 includes the apparatus of any of clauses 1-3, wherein the avatar position analyzer is to execute a neural network model to determine the first ergonomic form.
Claus 5 includes the apparatus of any of clauses 1-4, wherein the first ergonomic form includes a position of a body part of the user.
Clause 6 includes the apparatus of any of clauses 1-5, wherein the first ergonomic form includes a muscle tension level.
Clause 7 includes the apparatus of any of clauses 1-6, wherein the sensor data includes strain sensor data.
Clause 8 includes the apparatus of any of clauses 1-7, wherein the feedback generator is to cause the output device or a second output device to output haptic feedback in response to the determination of the second form.
Claus 9 includes the apparatus of any of clauses 1-8, wherein the feedback generator is to perform a comparison the first ergonomic form to the second form and cause a graphical feature of the avatar to be adjusted based on the comparison.
Clause 10 includes the apparatus of any of clauses 1-9, wherein the graphical feature includes a color of the avatar.
Clause 11 includes the apparatus of any of clauses 1-10, wherein the feedback generator is to cause the output device to display the graphical representation of the user as overlaying the avatar.
Clause 12 includes a system including a first sensor; and an extended reality coach controller to execute a neural network model to generate an avatar illustrating a first ergonomic position for a movement; cause an extended reality device to output the avatar; determine a second position of a body part of a user based on first sensor data generated by the first sensor; perform a comparison of the first ergonomic position and the second position; and cause the extended reality device to output graphical feedback based on the comparison.
Clause 13 includes the system of clause 12, wherein the first sensor includes an image sensor and the first sensor data includes image data including the user.
Clause 14 includes the system of clauses 12 or 13, wherein the extended reality coach controller is to generate the avatar based on a property of the user.
Clause 15 includes the system of any of clauses 12-14, wherein the graphical feedback includes a graphical representation of the user in the second position.
Clause 16 includes the system of any of clauses 12-15, wherein the extended reality coach controller is to cause the extended reality device to output the graphical representation of the user as overlaying an image of the avatar.
Clause 17 includes the system of any of clauses 12-16, wherein the extended reality coach controller is to cause a haptic feedback actuator to generate a haptic feedback output based on the comparison.
Clause 18 includes a non-transitory computer readable medium comprising instructions that, when executed by at least one processor, cause the at least one processor to generate an avatar based one or more properties of a user; determine a first ergonomic form for a movement based on the one or more properties of the user; cause an output device to display the avatar in the first ergonomic form; determine a second form associated with movement of the user based on sensor data collected via one or more sensors associated with the user; generate a graphical representation of the user in the second form; and cause the output device to display the graphical representation of the user with the avatar.
Clause 19 includes the non-transitory computer readable medium of clause 18, wherein the sensor data includes position data for one or more body parts of the user.
Clause 20 includes the non-transitory computer readable medium of clauses 18 or 19, wherein the sensor data includes image data including the user.
Clause 21 includes the non-transitory computer readable medium of any of clauses 18-20, wherein the instructions, when executed, cause the at least one processor to execute a neural network model to determine the first ergonomic form.
Clause 22 includes the non-transitory computer readable medium of any of clauses 18-21, wherein the first ergonomic form includes a position of a body part of the user.
Clause 23 includes the non-transitory computer readable medium of any of clauses 18-22, wherein the instructions, when executed, cause the at least one processor to cause the output device or a second output device to output haptic feedback in response to the determination of the second form.
Clause 24 includes the non-transitory computer readable medium of any of clauses 18-23, wherein the instructions, when executed, cause the at least one processor to perform a comparison the first ergonomic form to the second form and cause a graphical feature of the avatar to be adjusted based on the comparison.
Clause 25 includes the non-transitory computer readable medium of any of clauses 18-24, wherein the graphical feature includes a color of the avatar.
Clause 26 includes the non-transitory computer readable medium of any of clauses 18-25, wherein the instructions, when executed, cause the at least one processor to cause the output device to display the graphical representation of the user as overlaying the avatar.
Clause 27 includes a method including generating an avatar based one or more properties of a user; determining a first ergonomic form for a movement based on the one or more properties of the user; causing an output device to display the avatar in the first ergonomic form; determining a second form associated with movement of the user based on sensor data collected via one or more sensors associated with the user; generating a graphical representation of the user in the second form; and causing the output device to display the graphical representation of the user with the avatar.
Clause 28 includes the method of clause 27, wherein the sensor data includes position data for one or more body parts of the user.
Clause 29 includes the method of clauses 27 or 28, wherein the sensor data includes image data including the user.
Clause 30 includes the method of any of clauses 27-29, wherein determining the first ergonomic form includes executing a neural network model to determine the first ergonomic form.
Clause 31 includes the method of any of clauses 27-30, wherein the first ergonomic form includes a position of a body part of the user.
Clause 32 includes the method of any of clauses 27-31, further including causing the output device or a second output device to output haptic feedback in response to the determination of the second form.
Clause 33 includes the method of any of clauses 27-32, further including performing a comparison the first ergonomic form to the second form and causing a graphical feature of the avatar to be adjusted based on the comparison.
Clause 34 includes the method of any of clauses 27-33, wherein the graphical feature includes a color of the avatar.
Clause 35 includes the method of any of clauses 27-34, further including causing the output device to display the graphical representation of the user as overlaying the avatar.
Clause 36 includes a system including a first sensor; and an extended reality coach controller configured: to execute a neural network model to generate an avatar illustrating a first ergonomic position for a movement; to cause an extended reality device to output the avatar; to determine a second position of a body part of a user based on first sensor data generated by the first sensor; to perform a comparison of the first ergonomic position and the second position; and to cause the extended reality device to output graphical feedback based on the comparison.
Clause 37 includes the system of clause 36, wherein the first sensor includes an image sensor and the first sensor data include image data including the user.
Clause 38 includes the system of clauses 36 or 37, wherein the extended reality coach controller is configured to generate the avatar based on a property of the user.
Clause 39 includes the system of any of clauses 36-38, wherein the graphical feedback includes a graphical representation of the user in the second position.
Clause 40 includes the system of any of clauses 36-39, wherein the extended reality coach controller is configured to cause the extended reality device to output the graphical representation of the user as overlaying an image of the avatar.
Clause 41 includes the system of any of clauses 36-40, wherein the extended reality coach controller is configured to cause a haptic feedback actuator to generate a haptic feedback output based on the comparison.

Although certain example methods, apparatus and articles of manufacture have been disclosed herein, the scope of coverage of this patent is not limited thereto. On the contrary, this patent covers all methods, apparatus and articles of manufacture fairly falling within the scope of the claims of this patent.

## Claims

1. An apparatus comprising:
an avatar generator (216) configured to generate an avatar (300, 402) based on one or more properties of a user (102, 304);
an avatar position analyzer (214) configured to determine a first ergonomic form for a movement based on the one or more properties of the user (210), the avatar generator configured to cause an output device (104) to display the avatar in the first ergonomic form; and
a feedback generator (238) configured:
to determine a second form associated with movement of the user based on sensor data (200, 202, 204) collected via one or more sensors (112, 116, 118) associated with the user;
to generate a graphical representation (242) of the user in the second form; and
to cause the output device to display the graphical representation of the user with the avatar.

2. The apparatus of claim 1, wherein the sensor data include one or more of:
position data (200) for one or more body parts of the user;
image data (204) including the user; and
strain sensor data (202).

3. The apparatus of claim 1 or 2, wherein the avatar position analyzer is configured to execute a neural network model (234) to determine the first ergonomic form.

4. The apparatus of any of claims 1-3, wherein the first ergonomic form includes a position of a body part of the user.

5. The apparatus of any of claims 1-4, wherein the feedback generator is configured:
to cause the output device or a second output device (114, 124) to output haptic feedback in response to the determination of the second form, and/or wherein the feedback generator is to perform a comparison the first ergonomic form to the second form and cause a graphical feature of the avatar to be adjusted based on the comparison, wherein the graphical feature optionally includes a color of the avatar; and/or
to cause the output device to display the graphical representation of the user as overlaying the avatar.

6. A method comprising:
generating an avatar (300, 402) based one or more properties (210) of a user (102, 304);
determining a first ergonomic form for a movement based on the one or more properties of the user;
causing an output device (104) to display the avatar in the first ergonomic form;
determining a second form associated with movement of the user based on sensor data (200, 202, 204) collected via one or more sensors (112, 116, 118) associated with the user;
generating a graphical representation (242) of the user in the second form; and
causing the output device to display the graphical representation of the user with the avatar.

7. The method of claim 6, wherein the sensor data include position data (200) for one or more body parts of the user, and/or wherein the sensor data include image data (204) including the user.

8. The method of claim 6 or 7, wherein determining the first ergonomic form includes executing a neural network model (238) to determine the first ergonomic form.

9. The method of any of claims 6-8, wherein the first ergonomic form includes a position of a body part of the user.

10. The method of any of claims 6-9, further including causing the output device or a second output device (114, 124) to output haptic feedback in response to the determination of the second form.

11. The method of any of claims 6-10, further including performing a comparison the first ergonomic form to the second form and causing a graphical feature of the avatar to be adjusted based on the comparison.

12. The method of claim 11, wherein the graphical feature includes a color of the avatar.

13. The method of any of claims 6-12, further including causing the output device to display the graphical representation of the user as overlaying the avatar.

14. A computer program comprising computer program instructions that, when executed by a computer processor, cause the computer processor to perform the method of any of claims 6-13.

15. A non-transitory computer readable medium having stored thereon the computer program of claim 14.
